# EUROPEAN PATENT APPLICATION

(11) **EP 2 767 544 A1**
(43) Date of publication of application: **20.08.2014**
(21) Application number: 12817764.9
(22) Date of filing: 20.07.2012
(51) Int. Cl.: C07J 41/00

(54) **AMORPHOUS SUBSTANCE OF 17 -ACETOXY-11 -(4-N,N-DIMETHYLAMINOPHENYL)-19-NORPREGNA-4,9-DIENE-3,20-DIONE AND PREPARATION METHOD THEREOF**

(30) Priority: 22.07.2011 CN 201110207824
(71) Applicant: Shanghai Cdymax Pharmaceuticals Co., Ltd., Shanghai 201203 (CN); Jiangsu Cdymax Pharmaceuticals Co., Ltd., Jiangsu 226200 (CN)
(72) Inventor: AN, Xiaoxia, Shanghai 201203 (CN); LV, Feng, Shanghai 201203 (CN); SHEN, Shuxia, Shanghai 201203 (CN)
(74) Representative: Kling, Simone
(86) International application number: PCT/CN2012/078908
(87) International publication number: WO 2013/013594

(57) **Abstract**

The invention discloses an amorphous 17a-acetoxy-11β-(4-N,N-dimethylaminophenyl)-19-norpregna-4,9-diene-3,20-dione and a preparation method thereof. The amorphous CDB-2914 has an X-ray powder diffraction pattern basically shown as Figure 1. The preparation of the product comprises: provide a solution of CDB-2914 raw material in an organic solvent; in the presence of an anti-organic solvent, at a certain temperature, crystallize, thereby obtaining an amorphous CDB-2914.

## Description

### Technical field

The present invention relates to an amorphous 17α-acetoxy-11β-(4-N,N-dimethylaminophenyl)-19-norpregna-4,9-diene-3,20-dione (CDB-2914) and preparation method thereof.

### Background

CDB-2914 (Uliprisnil acetate) has a molecular formula of C₃₀H₃₇NO₄, a molecular weight of 475.634, CAS No. of 126784-99-4, and chemical name of 17α-acetoxy-11β-(4-N,N-dimethylaminophenyl)-19-norpregna-4,9-diene-3,20-dione, and its chemical structure is as follow:

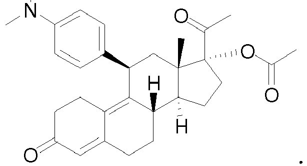

CDB-2914 is an emergency contraception drug, jointly developed by the U.S. National Institute of Child Health and Human Development and French HRA pharmaceutical company. CDB-2914 (trade name ELLA) was approved in Europe in May 2009 and firstly marketed in the UK, Germany and France on October 1. On August 13, 2010, the U.S. Food and Drug Administration (FDA) approved the application for marketing ELLA.

Patent US4/954,490 discloses the compound CDB-2914 and the synthesis method thereof.

Patent US5/929,262 discloses another synthesis method for CDB-2914. In this patent, the final product obtained by the method described in Example 7 was described as a product in yellow crystal form (referred to as crystal form A) with a melting point between 183 and 185°C.

Chinese patent 1753905 discloses a crystal form of CDB-2914 isopropanol hemisolvate (referred to as crystal form B) and its preparation method. the DSC of the isopropanol hemisolvate crystal form shows a characteristic absorption peak at 156°C. The 2θ value of the characteristic peaks and their corresponding intensity (%) of isopropanol hemisolvate crystal form are shown in Table 1:

**Table 1**

| 2θ(°) | Relative Intensity (%) |
|---|---|
| 9.085 | 100.0 |
| 8.860 | 56.1 |
| 16.375 | 53.7 |
| 17.750 | 49.3 |
| 18.720 | 44.2 |

In addition, CN1753905 also discloses are XRD and DSC data of CDB-2914 crystal form A in US 5929262. The DSC of this crystal form A shows a characteristic absorption peak at 189 °C. The 2θ value of the characteristic peaks and their corresponding intensity (%) of crystal form A are shown in Table 2:

**Table 2**

| 2θ(°) | Relative Intensity (%) |
|---|---|
| 9.110 | 100.0 |
| 16.965 | 64.1 |
| 15.130 | 41.3 |
| 15.010 | 41.2 |
| 17.165 | 39.2 |

The crystal form B (isopropanol hemisolvate) is a solvate, with a poor thermal stability; the crystal form A has a lower solubility and poor stability.

### Summary of Invention

To overcome the above-mentioned deficiencies existing in the prior art, an object of the invention is to provide an amorphous 17α-acetoxy-11β-(4-N,N-dimethylaminophenyl)-19-norpregna-4,9-diene-3,20-dione (CDB-2914) and its preparation method.

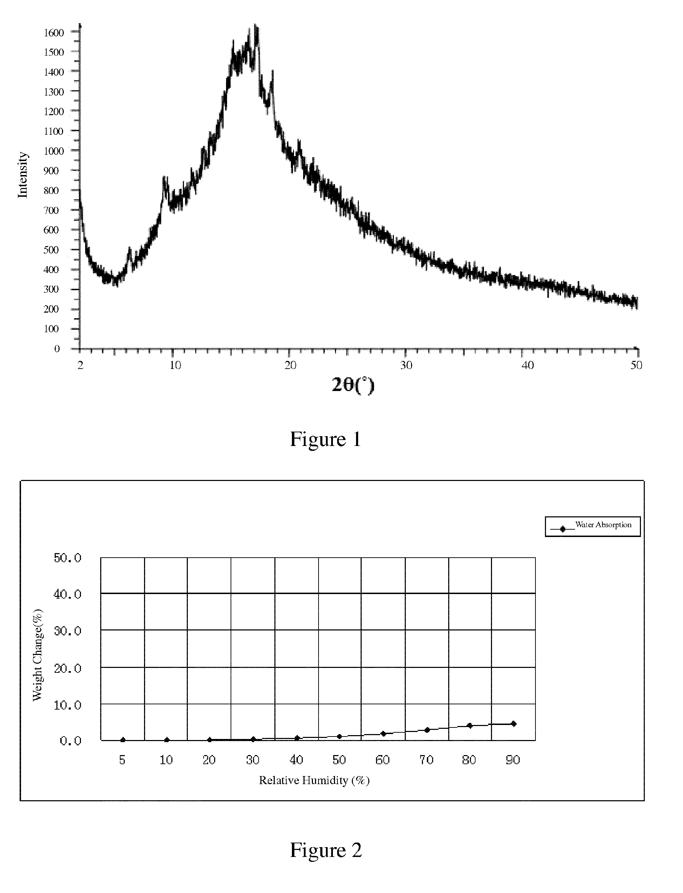

The amorphous CDB-2914 of the present invention has X-ray powder diffraction pattern basically as shown in figure 1.

A method for preparing the amorphous CDB-2914 comprises the following steps:
a) dissolve CDB-2914 raw material in an organic solvent at a dissolving temperature of 50 ∼ 78°C(preferably 50 ∼ 65°C);
b) add an anti-solvent when the CDB-2914 raw material is completely dissolved;
c) when a solid is precipitated, the mixture is stirred at the same temperature for 2 to 4 hours;
d) filter and dry.

The CDB-2914 raw material is any known crystal form.

The ratio of the CDB-2914 raw material and the organic solvent is that 1g of CDB-2914 raw material use 5 ∼ 15ml of organic solvent.

The amount of anti-solvent added to 1g of CDB-2914 raw material is 2 ∼ 30 ml.

The organic solvent is a routine organic solvent, such as methanol, ethanol, n-butanol, isopropanol, ethyl acetate and the like; preferably, methanol.

The anti-solvent is water, n-hexane, n-heptane, diisopropyl ether, methyl tert-butyl ether etc., preferably, water.

The amorphous CDB-2914 is prepared as the following method, comprising steps: provide a solution of CDB-2914 raw material in an organic solvent, and crystallize in the presence of an anti-solvent, at 50 ∼ 78°C (preferably, 50 ∼ 65°C), thereby obtaining an amorphous CDB-2914.

In another preferred embodiment, the organic solvent is methanol, ethanol, n-butanol, isopropanol or ethyl acetate; preferably, methanol or ethanol;
and/or the anti-solvent is water, n-hexane, n-heptane, diisopropyl ether or methyl tert-butyl ether, preferably, water.

In another preferred embodiment, the ratio of the CDB-2914 raw material and the organic solvent is that 1g of CDB-2914 raw material use 5 ∼ 15ml of organic solvent;
and/or the amount of anti-solvent added to 1g of CDB-2914 raw material is 2 ∼ 30 ml.

In another preferred embodiment, the purity of the amorphous CDB-2914 ≥ 95%; preferably ≥99 %.

A pharmaceutical composition of the invention comprises:
(a) the amorphous CDB-2914 of the invention; and
(b) a pharmaceutically acceptable carrier or excipient.

The amorphous CDB-2914 provided by the present invention has advantages such as good solubility, thermal stability and low moisture absorption, etc., and is more suitable for preparing a pharmaceutical preparation. Furthermore, the preparation method provided by the present invention has advantages such as high yield (quality yield of up to more than 90 %), high purity (HPLC purity of up to more than 99%), easy operation and implementation in large scale, etc., and meets requirements for industrial production.

### DESCRIPTION OF FIGURES

Figure 1 is XRPD of the amorphous CDB-2914 of the present invention.
Figure 2 is a hygroscopic pattern of the amorphous CDB-2914 of the present invention.

### Detailed Description of Invention

The invention will be illustrated in detail further accompanying the following drawings and embodiments.

### Example 1

To 5.0g CDB-2914 raw material (crystal form A ) was added 30mL of methanol, heated to 65 °C, and stirred till the solid completely dissolved; when the CDB-2914 raw material was completely dissolved, add 100ml water slowly; when a solid was precipitated, the mixture was stirred at 65 °C for 4 hours continuously; filtered and dried under reduced pressure at 60 °C for 12h, to give 4.7g product, quality yield 94%, HPLC purity 99.0%.

Take a sample of the amorphous CDB-2914 obtained in present example, and X-ray powder diffraction (XRPD), detected using Dedye-Scherrer INEL CPS-120 having a radiation source at a wavelength α1 of 1.5460 angstrom (Å) and a wavelength α2 of 1.54439 angstrom (Å), and a intensity ratio α1/α2 of 0.5, 40 kV voltage and 30mA current intensity was as shown in Figure 1. Figure 1 showed that: in XRPD of the obtained amorphous CDB-2914 there was no identified diffraction peak.

### Example 2

To 5.0g CDB-2914 raw material (isopropanol hemisolvate) was added 30mL of methanol, heated to 65 °C, and stirred till the solid completely dissolved; when the CDB-2914 raw material was completely dissolved, add 100ml water slowly; when a solid was precipitated, the mixture was stirred at 65 °C for 4 hours continuously; filtered and dried under reduced pressure at 60 °C for 12h, to give 4.6g product, quality yield 92%, HPLC purity 99.1%.

The XRPD of amorphous CDB-2914 obtained in the present example was shown as figure 1.

### Example 3

To 2.0g CDB-2914 raw material (crystal form A) was added 30mL of methanol, heated to 50 °C, and stirred till the solid completely dissolved; when the CDB-2914 raw material was completely dissolved, add 60ml water slowly; when a solid was precipitated, the mixture was stirred at 50 °C for 3 hours continuously; filtered and dried under reduced pressure at 60 °C for 12h, to give 1.8 g product, quality yield 90%, HPLC purity 99.0%.

The XRPD of amorphous CDB-2914 obtained in the present example was shown as figure 1.

### Example 4

To 4.0g CDB-2914 raw material (isopropanol hemisolvate) was added 20mL of methanol, heated to 60 °C, and stirred till the solid completely dissolved; when the CDB-2914 raw material was completely dissolved, add 60ml water slowly; when a solid was precipitated, the mixture was stirred at 60 °C for 2 hours continuously; filtered and dried under reduced pressure at 60 °C for 12h, to give 3.6 g product, quality yield 90%, HPLC purity 99.1%.

The XRPD of amorphous CDB-2914 obtained in the present example was shown as figure 1.

### Example 5

To 5.0g CDB-2914 raw material (crystal form A) was added 50mL of ethanol, heated to 78 °C, and stirred till the solid completely dissolved; when the CDB-2914 raw material was completely dissolved, add 100ml isopropyl ether slowly; when a solid was precipitated, the mixture was cooled to 50 °C naturally and stirred at the same temperature for 4 hours continuously; filtered and dried under reduced pressure at 60 °C for 12h, to give 4.62 g product, quality yield 92.4%, HPLC purity 99.0%.

The XRPD of amorphous CDB-2914 obtained in the present example was shown as figure 1.

### Example 6

To 2.0g CDB-2914 raw material (crystal form A) was added 20mL of iso-propanol, heated to 60 °C, and stirred till the solid completely dissolved; when the CDB-2914 raw material was completely dissolved, add 30ml n-hexane slowly; when a solid was precipitated, the mixture was stirred at 60 °C for 4 hours continuously; filtered and dried under reduced pressure at 60 °C for 12h, to give 1.83 g product, quality yield 91.5%, HPLC purity 99.1%.

The XRPD of amorphous CDB-2914 obtained in the present example was shown as figure 1.

### Example 7

To 2.0g CDB-2914 raw material (isopropanol hemisolvate) was added 30mL of n-butanol, heated to 70 °C, and stirred till the solid completely dissolved; when the CDB-2914 raw material was completely dissolved, add 30ml tert-butyl methyl ether slowly; when a solid was precipitated, the mixture was stirred at 70 °C for 4 hours continuously; filtered and dried under reduced pressure at 60 °C for 12h, to give 1.81 g product, quality yield 90.5%, HPLC purity 99.2%.

The XRPD of amorphous CDB-2914 obtained in the present example was shown as figure 1.

### Example 8

To 2.0g CDB-2914 raw material (isopropanol hemisolvate) was added 20mL of ethyl acetate, heated to 65 °C, and stirred till the solid completely dissolved; when the CDB-2914 raw material was completely dissolved, add 40ml n-hexane slowly; when a solid was precipitated, the mixture was stirred at 65 °C for 4 hours continuously; filtered and dried under reduced pressure at 60 °C for 12h, to give 1.80 g product, quality yield 90.0%, HPLC purity 99.3%.

The XRPD of amorphous CDB-2914 obtained in the present example was shown as figure 1.

### Example 9

To 2.0g CDB-2914 raw material (crystal form A) was added 30mL of ethyl acetate, heated to 60 °C, and stirred till the solid completely dissolved; when the CDB-2914 raw material was completely dissolved, add 30ml isopropyl ether slowly; when a solid was precipitated, the mixture was stirred at 60 °C for 4 hours continuously; filtered and dried under reduced pressure at 60 °C for 12h, to give 1.82 g product, quality yield 91.0%, HPLC purity 99.1%.

The XRPD of amorphous CDB-2914 obtained in the present example was shown as figure 1.

### Example 10: Solubility test

Add excess amount of known crystal (Crystal form A) and the amorphous substance sample of the present invention (prepared in Example 1-9) into 0.5ml of water, respectively, and then disperse and dissolve them by ultrasonic operation for several minutes (approximately 3 minutes). After standing for 30 minutes at room temperature, separate the supernatant by centrifugation. Determine the concentration of sample in supernatant by HPLC (defined as apparent solubility). The results were shown in Table 3.

**Table 3: Results of solubility determination**

| Sample | apparent solubility(mg/ml) |
|---|---|
| amorphous form | 0.001 |
| Known crystal(crystal form A) | 0.000125 |

It can be seen in table 3 that the amorphous CDB-2914 has better solubility compared to the known CDB-2914 crystal (crystal form A). The solubility of the amorphous CDB-2914 was 8 times of that of crystal form A.

### Example 11: Stability test

Dry the samples of the amorphous CDB-2914 obtained in the above-mentioned examples under reduced pressure at 80 °C for 8 hours, and then dry in vacuum for 15 hours. By analyzing XRPD, DSC, TGA, and IR it can be seen that the heat-treated product remained amorphous, and the XRPD is identical to figure 1, indicating that the amorphous CDB-2914 obtained in the present invention is thermally stable.

### Example 12: Hygroscopicity test

Evaluate hygroscopicity of the amorphous substance using a microbalance (MBA300W, WTI Company, USA). Hang the sample placed on the glass rack in an apparatus that preconditioned at 25 °C, and track the changes of sample weight at a relative humidity of 5% to 95%. Measure the weight of sample at each measurement point (relative humidity) at 2 minutes intervals. The final value was the weight unit at a time point that the variability is less than 0.2%.

Figure 2 is the hygroscopic pattern of the amorphous CDB-2914 prepared in the present patent. It can be seen in Figure 2 that the amorphous CDB-2914 has a low hygroscopicity.

### Example 13 Pharmaceutical composition

Weigh 100 g of amorphous CDB-2914 prepared in Examples 1 to 9, mixed well with 4 times-weighted starch, and formulated into tablets according to a conventional method.

## Claims

1. An amorphous 17α-acetoxy-11β-(4-N,N-dimethylaminophenyl)-19-norpregna-4,9-diene-3,20-dione (CDB-2914) has a X-ray powder diffraction pattern basically as shown in figure 1.

2. A method for preparing the amorphous CDB-2914 of claim 1 comprises the following steps:
a) dissolve CDB-2914 raw material in an organic solvent at a dissolving temperature of 50 ∼ 78°C;
b) add an anti-solvent when the CDB-2914 raw material is completely dissolved;
c) when a solid is precipitated, the mixture is stirred at the same temperature for 2 to 4 hours;
d) filter and dry.

3. The method for preparing the amorphous CDB-2914 of claim 2, wherein, the CDB-2914 raw material is any known crystal form.

4. The method for preparing the amorphous CDB-2914 of claim 2, wherein, the dissolving temperature is 50 ∼ 65 °C.

5. The method for preparing the amorphous CDB-2914 of claim 2, wherein, the ratio of the CDB-2914 raw material and the organic solvent is that 1g of CDB-2914 raw material use 5 ∼ 15ml of organic solvent.

6. The method for preparing the amorphous CDB-2914 of claim 2, wherein, the amount of anti-solvent added to 1g of CDB-2914 raw material is 2 ∼ 30 ml.

7. The method for preparing the amorphous CDB-2914 of claim 2, wherein, the organic solvent is methanol, ethanol, n-butanol, isopropanol, ethyl acetate.

8. The method for preparing the amorphous CDB-2914 of claim 2, wherein, the anti-solvent is water, n-hexane, n-heptane, diisopropyl ether, methyl tert-butyl ether.

9. An amorphous CDB-2914 is prepared as the following method, comprising steps:
provide a solution of CDB-2914 raw material in an organic solvent, and crystallize in the presence of an anti-solvent, at 50 ∼ 78°C, thereby obtaining an amorphous CDB-2914.

10. A pharmaceutical composition, wherein, it comprises:
(a) the amorphous CDB-2914 of claim 1 or 9; and
(b) a pharmaceutically acceptable carrier or excipient.
